# EUROPEAN PATENT SPECIFICATION

(11) **EP 3 287 141 B1**
(45) Date of publication and mention of the grant of the patent: **18.08.2021**
(21) Application number: 16782602.3
(22) Date of filing: 18.04.2016
(51) Int. Cl.: A61K 38/18, A61K 9/08, A61K 9/19, A61P 25/00

(54) **NERVE GROWTH FACTOR COMPOSITION AND INJECTION POWDER**
NERVENWACHSTUMSFAKTORZUSAMMENSETZUNG UND INJEKTIONSPULVER
COMPOSITION DE FACTEUR DE CROISSANCE NERVEUX ET POUDRE INJECTABLE

(30) Priority: 21.04.2015 CN 201510190675
(43) Date of publication of application: 28.02.2018
(73) Proprietor: Staidson (Beijing) Biopharmaceuticals Co., Ltd., Beijing 100176 (CN); Beijing Staidson Medical Technology Co., Ltd, Beijing 100176 (CN)
(72) Inventor: TAN, Jianping, Beijing 100176 (CN); WANG, Bingzhang, Beijing 100176 (CN)
(74) Representative: ABG Intellectual Property Law, S.L.
(86) International application number: PCT/CN2016/079572
(87) International publication number: WO 2016/169455

(56) References cited:
- WO-A1-95/05845
- WO-A1-2007/073035
- WO-A1-2013/063510
- CN-A- 1 552 440
- CN-A- 1 552 440
- CN-A- 1 698 883
- CN-A- 1 824 297
- CN-A- 101 972 224
- CN-A- 101 972 470
- CN-A- 102 512 664
- CN-A- 104 023 748
- US-A1- 2008 112 953

## Description

### Technical Field

The present invention relates to a nerve growth factor composition and a powder for injection, and belongs to the field of pharmaceutical biology.

### Background Art

Nerve growth factor (NGF) is a nerve cell growth regulator with the double biological functions of neuronal nutrition and neurite growth promotion, and it has an important regulatory effect on the development, differentiation, growth, regeneration and functional property expression of the central and peripheral neurons. NGF contains three subunits, α, β, γ, wherein the β subunit is an active region, formed by two single chains composed of 118 amino acids by a non-covalent bond. In 1953, the Italian scientist Levi-Montalcini discovered NGF and won the Nobel Prize. At present, there are a number of NGF products which appear on the market all over the world, and they are clinically used mainly for the treatment of neurological dysplasia, including amblyopia, neuroma, various nerve injury and neurological diseases and other diseases.

NGF is similar to other protein drugs; since the protein has a short half life, the spatial conformation of the protein easily changes and thus results in protein denaturation when exposed to extreme temperature and humidity conditions, or by influenced by physical and chemical factors; the denaturated protein will lose its original biological activity; in addition, because the protein often tends to adhere to a solid surface, in the filling process, part of the protein will adhere to the inner wall of the container, resulting in the loss of active ingredients. In order to ensure its biological activity, there is a need to add a stabilizer to prevent the loss of the biological activity.

Generally, albumin is widely used in various biological products as an excellent stabilizer and as a cake forming agent. WO 95/05845 A1 discloses an NGF composition comprising human serum albumin as stabilizer. However, since albumin is mainly derived from human blood, placental blood, and the blood may carry some of the unknown components which are not easy to be detected, the NGF composition as a non-sterile preparation which is sterilized, may easily be contaminated; at the same time, the long-term and wide applications of albumin are also susceptible to blood supply constraints and production costs; again, in the determination of the contents of the intermediates and preparation of finished product, albumin may interfere with a relatively small amount of NGF and thus affect the product quality management. Therefore, in order to avoid the above problems, it is necessary to find a stable albumin-free NGF composition. In this regard, CN 1 552 440 A and CN 101 972 470 A disclose the use of mixtures of amino as stabilizers for NGF compositions. US 2008/112953 A1 discloses polypeptide/antibody compositions comprising a variety of excipients including amino acids. However, these documents are silent about the use of an amino acid mixture comprising isoleucine for stabilization purposes.

### Summary of the Invention

It is an object of the present invention to provide a nerve growth factor composition and a powder for injection which not only protect mouse-derived nerve growth factor (mNGF) but also can ensure the good stability of human-derived nerve growth factor (hNGF) and recombinant human nerve growth factor (rhNGF) in the preparation, transportation and storage processes, and have better clinical medication safety and quality controllability.

The present invention provides a nerve growth factor composition comprising a nerve growth factor, a stabilizer, a supporting agent, a pH buffer and a water as defined in claim 1; said stabilizer is arginine, glutamic acid, glycine and isoleucine, and said supporting agent is any one of mannitol, dextrin and sorbitol.

In the above nerve growth factor composition:
a mass-volume concentration of said nerve growth factor is 10 µg/mL-100 µg/mL;
a mass-volume concentration of said stabilizer is 6 mg/mL-30 mg/mL;
a mass-volume concentration of said supporting agent is 20 mg/mL-50 mg/mL;
said pH buffer maintains said nerve growth factor composition at a pH value of 6.80 to 7.00;
solvent is water.

The addition of a stabilizer in the above nerve growth factor composition avoids or reduces the aggregation and depolymerization of proteins caused in the preparation or storage process, and the term "stabilizer" refers to a substance which prevents the active ingredient from aggregating or depolymerizing in an aqueous solution, and in addition to the function of stability, the stabilizer can also be used as a supporting agent to improve the product formability, with other functions being not excluded;

In the above nerve growth factor composition, preferably, the mass-volume concentration of said stabilizer is 10 mg/mL-20 mg/mL, and more preferably, the mass-volume concentration of said stabilizer is 13 mg/mL;

In the above nerve growth factor composition, the mass ratio of arginine to glutamic acid to glycine to isoleucine in said stabilizer is 1 : (1-3) : (1-3) : (1-3.6), specifically may be 1 : (1-1.3) : (1-1.8) : (1-1.3), 1 : (1.3-3) : (1.8-3.0) : (1.3-3.6), 1 : (1.2-2) : (1.5-2.0) : (1.2-1.5), 1 : (1.25-2.8) : (1.6-2.8) : (1.25-3.0), 1 : 1 : 1 : 1 : 1 : 1.2 : 3 : 1.3, 1 : 2 : 3 : 1,1 : 1.25 : 1.5 : 1.25, 1 : 1 : 2.8 : 1.5, 1 : 2 : 2 : 3.6, 1 : 1.3 : 3 : 1.3, 1 : 3 : 3: 3, 1 : 1.2 : 1.6 : 1.2 or 1 : 2.8 : 1.8 : 1.5, preferably, the mass ratio of arginine to glutamic acid to glycine to isoleucine in said stabilizer is 1 : 1.25 : 1.5 : 1.25.

In the above nerve growth factor composition, the addition of the supporting agent can improve the appearance of the finished product and ensure that the product has good formability after lyophilization;
in the above nerve growth factor composition, preferably, the mass-volume concentration of said supporting agent is 30 mg/mL-50 mg/mL, and more preferably, the mass-volume concentration of said supporting agent is 50 mg/mL;
in the present invention said supporting agent is any of mannitol, dextrin and sorbitol.

In the above nerve growth factor composition, in order to ensure that the composition has the greatest biological activity, it is generally necessary to control an optimum pH range. The optimum pH range for this stability needs to be determined at the time of formulation screening, usually using an influencing factor test (illumination, high temperature, high humidity), accelerated and long-term stability tests and other methods. After the determination of the formulation, the composition in the production and storage process must be maintained at its optimum pH range. Since the buffer has good buffering capacity, the relative stability of the product pH can be maintained in a certain range; therefore, a buffer is often added for the control of pH value in the formulation;
in the above nerve growth factor composition, the molar concentration of said pH buffer is 10 mM-50 mM, preferably, the molar concentration of said pH buffer is 20 mM-25 mM, and more preferably, the molar concentration of said pH buffer is 25 mM.

In the above nerve growth factor composition, preferably, said nerve growth factor composition is maintained at the pH value of 6.86-6.91, and more preferably, said nerve growth factor is maintained at the pH value of 6.82.

In the above nerve growth factor composition, said pH buffer is selected from one or more of a phosphate, a citrate, an acetate, a histidine hydrochloride and an arginine hydrochloride, specifically may be any one of the following 1)-5): 1) an arginine hydrochloride, 2) a phosphate, 3) a citrate, 4) an acetate, 5) a histidine hydrochloride and an arginine hydrochloride.

In the above nerve growth factor composition, said water is water for injection.

In the above nerve growth factor composition, said nerve growth factor is a nerve cell growth regulator with the double biological functions of neuronal nutrition and neurite growth promotion, and it has an important regulatory effect on the development, differentiation, growth, regeneration and functional property expression of the central and peripheral neurons;
in the above nerve growth factor composition, preferably, the mass-volume concentration of said nerve growth factor is 40 µg/mL-80 µg/mL, more preferably the mass-volume concentration of said nerve growth factor is 50 µg/mL.

In the above nerve growth factor composition, said nerve growth factor is selected from a mouse-derived nerve growth factor, a human-derived nerve growth factor, or a recombinant human nerve growth factor.

In the above nerve growth factor composition, the concentration of each component may be any one of the following 1) to 15):
1) the nerve growth factor: 10-50 µg/mL, the stabilizer: 6-13 mg/mL, the supporting agent: 20 mg/mL-40 mg/mL, the pH buffer: 10 mM-25 mM;
2) the nerve growth factor: 60-100 µg/mL, the stabilizer: 6.5-14 mg/mL, the supporting agent: 30 mg/mL-50 mg/mL, the pH buffer: 25 mM-50 mM;
3) the nerve growth factor: 40-80 µg/mL, the stabilizer: 8.9-15 mg/mL, the supporting agent: 30 mg/mL-50 mg/mL, the pH buffer: 20 mM-30 mM;
4) the nerve growth factor: 40-80 µg/mL, the stabilizer: 10-20 mg/mL, the supporting agent: 30 mg/mL-50 mg/mL, the pH buffer: 20 mM-25 mM;
5) the nerve growth factor: 50-80 µg/mL, the stabilizer: 10-20 mg/mL, the supporting agent: 40 mg/mL-50 mg/mL, the pH buffer: 20 mM-25 mM;
6) the nerve growth factor: 10 µg/mL, the stabilizer: 6 mg/mL, the supporting agent: 20 mg/mL, the pH buffer: 10 mM;
7) the nerve growth factor: 40 µg/mL, the stabilizer: 6.5 mg/mL, the supporting agent: 30 mg/mL, the pH buffer: 20 mM;
8) the nerve growth factor: 50 µg/mL, the stabilizer: 8.9 mg/mL, the supporting agent: 40 mg/mL, the pH buffer: 25 mM;
9) the nerve growth factor: 60 µg/mL, the stabilizer: 10 mg/mL, the supporting agent: 50 mg/mL, the pH buffer: 30 mM;
10) the nerve growth factor: 80 µg/mL, the stabilizer: 13 mg/mL, the supporting agent: 50 mg/mL, the pH buffer: 30 mM;
11) the nerve growth factor: 100 µg/mL, the stabilizer: 14 mg/mL, the supporting agent: 50 mg/mL, the pH buffer: 30 mM;
12) the nerve growth factor: 100 µg/mL, the stabilizer: 15 mg/mL, the supporting agent: 50 mg/mL, the pH buffer: 30 mM;
13) the nerve growth factor: 100 µg/mL, the stabilizer: 20 mg/mL, the supporting agent: 50 mg/mL, the pH buffer: 30 mM;
14) the nerve growth factor: 100 µg/mL, the stabilizer: 30 mg/mL, the supporting agent: 50 mg/mL, the pH buffer: 30 mM;
15) the nerve growth factor: 50 µg/mL, the stabilizer: 10 mg/mL, the supporting agent: 40 mg/mL, the pH buffer: 25 mM.

In the above composition of the nerve growth factor, the pH value of said nerve growth factor composition may be 6.80-7.00, specifically may be 6.80, 6.81, 6.83, 6.85, 6.91 or 7.00.

In the above nerve growth factor composition, since the protein has a high tendency to interact with a surface, it is susceptible to adsorption and/or denaturation at a gas-liquid, bottle-liquid interface, which is inversely proportional to the protein concentration, and leads to the formation of soluble and insoluble protein aggregates, or the loss of protein in the solution by adsorption to the interface.

A surfactant is often used in the protein formulation for preventing the adsorption and/or denaturation initiated by the surface interactions. The surfactant is an amphiphilic molecule that competes with protein at the interface position. The hydrophobic portion of the surfactant molecule occupies the interface position (e.g., gas-liquid), while the hydrophilic portion of the molecule remains directed to the solvent bulk. At a sufficient concentration (usually around the critical micelle concentration of the surfactant), the surface layer of the surfactant molecule acts to prevent the adsorption of protein molecules at the interface. In addition, in the whole process of lyophilization of biological products, the surfactant can not only reduce the freezing and dehydrating denaturations caused by the reduction in the interfacial tension of the ice water surface in the freezing and dehydrating processes, but also have the function of a wetting agent and a refolding agent on the active components in the rehydrating process.

The above nerve growth factor composition may also comprise a surfactant. The mass-volume concentration of said surfactant is 0 mg/mL-1.0 mg/mL, but not 0; preferably, the mass-volume concentration of said surfactant is 0.2 mg/mL-1.0 mg/mL; preferably, the mass-volume concentration of said surfactant is 0.2-0.5 mg/ml; more preferably, the mass-volume concentration of said surfactant is 0.2 mg/mL.

In the above nerve growth factor composition, said surfactant is any one of poloxamer, polysorbate, and 15-polyethleneglycol hydroxystearate (abbreviated as HS 15, the same below), preferably said surfactant is poloxamer 188 (abbreviated as F68), polysorbate 20 (abbreviated as TW-20), polysorbate 80 (abbreviated as TW-80) and HS 15.

The present invention further provides a method for preparing the nerve growth factor composition in the form of powder for injection, wherein the above nerve growth factor composition is lyophilized to obtain the nerve growth factor composition in the form of powder for injection.

The present invention also provides a nerve growth factor composition in the form of powder for injection for injection administration.

The water content of the above nerve growth factor composition in the form of powder for injection may be 1.0-3.0%, preferably 1.0-2.0%, specifically may be 1.1-1.9%, 1.1%, 1.3%, 1.5%, 1.6%, 1.7% or 1.9%.

The above nerve growth factor compositions for use in the treatment of nerve injury or the preparation of a medicament for the treatment of nerve injury is also within the scope of the present invention. Said nerve growth factor composition may specifically be a nerve growth factor composition in the form of powder for injection. The nerve injury may be an optic nerve injury, and the cause of the injury may be a fist injury, a car accident, a physical hit injury or an eye explosive injury.

A method of treating nerve injury comprising the step of: administering an effective amount of a nerve growth factor composition to a patient with the nerve injury is disclosed but not claimed herein. Said nerve growth factor composition may specifically be a nerve growth factor composition in the form of powder for injection. The administration may specifically be intramuscular injection. The nerve injury may be an optic nerve injury, and the cause of the injury may be a fist injury, a car accident, a physical hit injury or an eye explosive injury.

### Brief Description of the Drawings

Fig.1 shows the change curve of the average content of NGF over time in the NGF compositions in the form of powder for injection prepared in the Examples and the reference preparation, under the accelerated condition (25°C, RH 60 ± 10%).
Fig.2 shows the change curve of the average activity over time of the NGF compositions in the form of powder for injection prepared in the Examples and the reference preparation, under the accelerated condition (25°C, RH 60 ± 10%).
Fig.3 shows the change curve of the average content of NGF over time in the NGF compositions in the form of powder for injection prepared in the Examples and the reference preparation, under the long-term stability condition (6 ± 2°C).
Fig.4 shows the change curve of the average activity over time of the NGF compositions in the form of powder for injection prepared in the Examples and the reference preparation, under the long-term stability condition (6 ± 2°C).

### Detailed Description of Embodiments

The experimental methods used in the following Examples are conventional methods unless otherwise specified.

The materials, reagents and the like used in the following Examples are commercially available, unless otherwise specified.

The mNGF and hNGF stock solutions used in the following Examples are supplied by the Staidson (Beijing) Biopharmaceuticals Co., Ltd., the rhNGF stock solution is supplied by the Sino Biological Inc., and the rest of the excipients are all injection grade unless otherwise specified.

In order to find a substance with better stability as a stabilizer of NGF, in the present invention, a large number of exploratory experiments have been carried out by screening and optimizing the type and amount of amino acids, according to the structural property of NGF, and finally the different concentrations and proportions of glycine, arginine, glutamic acid and isoleucine are used to prepare the various sterile NGF composition in the form of powder for injection and the moisture, pH, osmotic pressure, content and activity of the finished product after lyophilization are determined.

### Example 1. Preparation of NGF composition and powder for injection

The NGF composition and the powder for injection are prepared as follows:
a formulation amount of a arginine hydrochloride is weighed according to the formulation composition in Table 1, an appropriate amount of water for injection is added and stirred to complete dissolution, then formulation amounts of glutamic acid, arginine, isoleucine, glycine, mannitol, and F68 are added and stirred to complete dissolution, a rhNGF stock solution is added, the pH is adjusted to pH 6.85 as shown in Table 1 with arginine or HCl, water for injection is added to the volume of the scale, after having been mixed uniformly, the mixture is filtered through a 0.22 µm microfiltration membrane into a sterile container to prepare a composition having an NGF concentration of about 10 µg/mL.

The above rhNGF composition is subpackaged into an injection bottle made from neutral borosilicate glass injection tube at 0.63 ± 0.03 mL/bottle and lyophilized, to prepare a sterile , i.e., a rhNGF composition in the form of powder for injection.

### Example 2. Preparation of NGF composition and powder for injection

The NGF composition and the powder for injection are prepared as follows:
a formulation amount of a phosphate is weighed according to the formulation composition in Table 1, an appropriate amount of water for injection is added and stirred to complete dissolution, then formulation amounts of glutamic acid, arginine, isoleucine, glycine, and mannitol are added and stirred to complete dissolution, a hNGF stock solution is added, the pH is adjusted to pH 6.83 as shown in Table 1 with NaOH or H₃PO₄, water for injection is added to the volume of the scale, after having been mixed uniformly, the mixture is filtered through a 0.22 µm microfiltration membrane into a sterile container to prepare a composition having an NGF concentration of about 40 µg/mL.

The above hNGF composition is subpackaged into an injection bottle made from neutral borosilicate glass injection tube at 0.63 ± 0.03 mL/bottle and lyophilized, to prepare a sterile powder for injection, i.e., a hNGF composition in the form of powder for injection.

### Example 3. Preparation of NGF composition and powder for injection

The NGF composition and the powder for injection are prepared as follows:
a formulation amount of a citrate is weighed according to the formulation composition in Table 1, an appropriate amount of water for injection is added and stirred to complete dissolution, then formulation amounts of glutamic acid, arginine, isoleucine, glycine, and mannitol are added and stirred to complete dissolution, a hNGF stock solution is added, the pH is adjusted to pH 6.80 as shown in Table 1 with NaOH or citric acid, water for injection is added to the volume of the scale, after having been mixed uniformly, the mixture is filtered through a 0.22 µm microfiltration membrane into a sterile container to prepare a composition having an NGF concentration of about 40 µg/mL.

The above hNGF composition is subpackaged into an injection bottle made from neutral borosilicate glass injection tube at 0.63 ± 0.03 mL/bottle and lyophilized, to prepare a sterile powder for injection, i.e., a hNGF composition in the form of powder for injection.

### Example 4. Preparation of NGF composition and powder for injection

The NGF composition and the powder for injection are prepared as follows:
a formulation amount of a phosphate is weighed according to the formulation composition in Table 1, an appropriate amount of water for injection is added and stirred to complete dissolution, then formulation amounts of glutamic acid, arginine, isoleucine, glycine, and mannitol are added and stirred to complete dissolution, a hNGF stock solution is added, the pH is adjusted to pH 6.91 as shown in Table 1 with NaOH or H₃PO₄, water for injection is added to the volume of the scale, after having been mixed uniformly, the mixture is filtered through a 0.22 µm microfiltration membrane into a sterile container to prepare a composition having an NGF concentration of about 80 µg/mL.

The above hNGF composition is subpackaged into an injection bottle made from neutral borosilicate glass injection tube at 0.63 ± 0.03 mL/bottle and lyophilized, to prepare a sterile powder for injection, i.e., a hNGF composition in the form of powder for injection.

### Example 5. Preparation of NGF composition and powder for injection

The NGF composition and the powder for injection are prepared as follows:
a formulation amount of an acetate is weighed according to the formulation composition in Table 1, an appropriate amount of water for injection is added and stirred to complete dissolution, then formulation amounts of glutamic acid, arginine, isoleucine, glycine, and mannitol are added and stirred to complete dissolution, a rhNGF stock solution is added, the pH is adjusted to pH 6.82 as shown in Table 1 with NaOH or HAc, water for injection is added to the volume of the scale, after having been mixed uniformly, the mixture is filtered through a 0.22 µm microfiltration membrane into a sterile container to prepare a composition having an NGF concentration of about 80 µg/mL.

The above rhNGF composition is subpackaged into an injection bottle made from neutral borosilicate glass injection tube at 0.63 ± 0.03 mL/bottle and lyophilized, to prepare a sterile powder for rhNGF injection, i.e., a rhNGF composition in the form of powder for injection.

### Example 6. Preparation of NGF composition and powder for injection

The NGF composition and the powder for injection are prepared as follows:
a formulation amount of a citrate is weighed according to the formulation composition in Table 1, an appropriate amount of water for injection is added and stirred to complete dissolution, then formulation amounts of glutamic acid, arginine, isoleucine, glycine, and sorbitol are added and stirred to complete dissolution, a hNGF stock solution is added, the pH is adjusted to pH 6.82 as shown in Table 1 with NaOH or citric acid, water for injection is added to the volume of the scale, after having been mixed uniformly, the mixture is filtered through a 0.22 µm microfiltration membrane into a sterile container to prepare a composition having an NGF concentration of about 50 µg/mL.

The above hNGF composition is subpackaged into an injection bottle made from neutral borosilicate glass injection tube at 0.63 ± 0.03 mL/bottle and lyophilized, to prepare a sterile powder for injection, i.e., a hNGF composition in the form of powder for injection.

### Example 7. Preparation of NGF composition and powder for injection

The NGF composition and the powder for injection are prepared as follows:
a formulation amount of an acetate is weighed according to the formulation composition in Table 1, an appropriate amount of water for injection is added and stirred to complete dissolution, then formulation amounts of glutamic acid, arginine, isoleucine, glycine and mannitol are added and stirred to complete dissolution; since the TW-80 in the formulation is in a spherical aggregate state in the water for injection and is not easily mixed uniformly, TW-80 is firstly dissolved in hot water for injection (40°C-80°C) to formulate into an 1% aqueous solution, and after having been cooled to room temperature, it is added into the above solution in an amount converted from the formulation amount, and mixed uniformly in the present invention; a hNGF stock solution is added, the pH is adjusted to pH 6.81 as shown in Table 1 with NaOH or HAc, and water for injection is add to the volume of the scale, after having been mixed uniformly, the mixture is filtered through a 0.22 µm microporous membrane filter into a sterile container to prepare a composition having an NGF concentration of about 60 µg/mL.

The above hNGF composition is subpackaged into an injection bottle made from neutral borosilicate glass injection tube at 0.63 ± 0.03 mL/bottle and lyophilized, to prepare a sterile powder for injection, i.e., a hNGF composition in the form of powder for injection.

### Example 8. Preparation of NGF composition and powder for injection

The NGF composition and the powder for injection are prepared as follows:
formulation amounts of a histidine hydrochloride and an arginine hydrochloride are weighed according to the formulation composition in Table 1, an appropriate amount of water for injection is added and stirred to complete dissolution, then formulation amounts of glutamic acid, arginine, isoleucine, glycine, and dextrin are added and stirred to complete dissolution, a mNGF stock solution is added, the pH is adjusted to pH 6.85 as shown in Table 1 with arginine or HCl, water for injection is added to the volume of the scale, after having been mixed uniformly, the mixture is filtered through a 0.22 µm microfiltration membrane into a sterile container to prepare a composition having an NGF concentration of about 60 µg/mL.

The above mNGF composition is subpackaged into an injection bottle made from neutral borosilicate glass injection tube at 0.63 ± 0.03 mL/bottle and lyophilized, to prepare a sterile powder for injection.

### Example 9. Preparation of NGF composition and powder for injection

The NGF composition and the powder for injection are prepared as follows:
formulation amounts of a histidine hydrochloride and an arginine hydrochloride are weighed according to the formulation composition in Table 1, an appropriate amount of water for injection is added and stirred to complete dissolution, then formulation amounts of glutamic acid, arginine, isoleucine, glycine, and mannitol are added and stirred to complete dissolution, a hNGF stock solution is added, the pH is adjusted to pH 6.83 as shown in Table 1 with arginine or HCl, water for injection is added to the volume of the scale, after having been mixed uniformly, the mixture is filtered through a 0.22 µm microfiltration membrane into a sterile container to prepare a composition having an NGF concentration of about 50 µg/mL.

The above hNGF composition is subpackaged into an injection bottle made from neutral borosilicate glass injection tube at 0.63 ± 0.03 mL/bottle and lyophilized, to prepare a sterile powder for injection, i.e., a hNGF composition in the form of powder for injection.

### Example 10. Preparation of NGF composition and powder for injection

The NGF composition and the powder for injection are prepared as follows:
a formulation amount of a phosphate is weighed according to the formulation composition in Table 1, an appropriate amount of water for injection is added and stirred to complete dissolution, then formulation amounts of glutamic acid, arginine, isoleucine, glycine, and mannitol are added and stirred to complete dissolution, a mNGF stock solution is added, the pH is adjusted to pH 7.00 as shown in Table 1 with NaOH or H₃PO₄, water for injection is added to the volume of the scale, after having been mixed uniformly, the mixture is filtered through a 0.22 µm microfiltration membrane into a sterile container to prepare a composition having an NGF concentration of about 100 µg/mL.

The above mNGF composition is subpackaged into an injection bottle made from neutral borosilicate glass injection tube at 0.63 ± 0.03 mL/bottle and lyophilized, to prepare a sterile powder for injection, i.e., a mNGF composition in the form of powder for injection.

### Example 11. Preparation of NGF composition and powder for injection

The NGF composition and the powder for injection are prepared as follows:
a formulation amount of an acetate is weighed according to the formulation composition in Table 1, an appropriate amount of water for injection is added and stirred to complete dissolution, then formulation amounts of glutamic acid, arginine, isoleucine, glycine, and mannitol are added and stirred to complete dissolution, a hNGF stock solution is added, the pH is adjusted to pH 6.83 as shown in Table 1 with NaOH or HAc, water for injection is added to the volume of the scale, after having been mixed uniformly, the mixture is filtered through a 0.22 µm microfiltration membrane into a sterile container to prepare a composition having an NGF concentration of about 60 µg/mL.

The above hNGF composition is subpackaged into an injection bottle made from neutral borosilicate glass injection tube at 0.63 ± 0.03 mL/bottle and lyophilized, to prepare a sterile powder for injection, i.e., a hNGF composition in the form of powder for injection.

### Example 12. Preparation of NGF composition and powder for injection

The NGF composition and the powder for injection are prepared as follows:
a formulation amount of a citrate is weighed according to the formulation composition in Table 1, an appropriate amount of water for injection is added and stirred to complete dissolution, then formulation amounts of glutamic acid, arginine, isoleucine, glycine and sorbitol are added and stirred to complete dissolution; since the HS 15 in the formulation is a solid block at normal temperature and is not easily mixed uniformly, HS 15 is firstly dissolved in hot water for injection (40°C-80°C) to formulate into an 1% aqueous solution, and after having been cooled to room temperature, it is added into the above solution in an amount converted from the formulation amount, and mixed uniformly in the present invention; a hNGF stock solution is added, the pH is adjusted to pH 6.85 as shown in Table 1 with NaOH or citric acid, and water for injection is add to the volume of the scale, after having been mixed uniformly, the mixture is filtered through a 0.22 µm microporous membrane filter into a sterile container to prepare a composition having an NGF concentration of about 50 µg/mL.

The above hNGF composition is subpackaged into an injection bottle made from neutral borosilicate glass injection tube at 0.63 ± 0.03 mL/bottle and lyophilized, to prepare a sterile powder for injection, i.e., a hNGF composition in the form of powder for injection.

### Example 13. Preparation of NGF composition and powder for injection

The NGF composition and the powder for injection are prepared as follows:
a formulation amount of a phosphate is weighed according to the formulation composition in Table 1, an appropriate amount of water for injection is added and stirred to complete dissolution, then formulation amounts of glutamic acid, arginine, isoleucine, glycine and mannitol are added and stirred to complete dissolution; since the TW-20 in the formulation is in a spherical aggregate state in the water for injection and is not easily mixed uniformly, TW-20 is firstly dissolved in hot water for injection (40°C-80°C) to formulate into an 1% aqueous solution, and after having been cooled to room temperature, it is added into the above solution in an amount converted from the formulation amount, and mixed uniformly in the present invention; a hNGF stock solution is added, the pH is adjusted to pH 6.82 as shown in Table 1 with NaOH or HAc, and water for injection is add to the volume of the scale, after having been mixed uniformly, the mixture is filtered through a 0.22µm microporous membrane filter into a sterile container to prepare a composition having an NGF concentration of about 50 µg/mL.

The above hNGF composition is subpackaged into an injection bottle made from neutral borosilicate glass injection tube at 0.63 ± 0.03 mL/bottle and lyophilized, to prepare a sterile powder for injection, i.e., a hNGF composition in the form of powder for injection.

**Table 1. The amounts of components in Examples 1-13**

| Formul ation compos ition | Use | Amounts of components in the Examples | | | | | | | | | | | | |
|---|---|---|---|---|---|---|---|---|---|---|---|---|---|---|
| | | Exa mpl e 1 | Exa mpl e 2 | Exa mpl e 3 | Exa mpl e 4 | Exa mpl e 5 | Exa mpl e 6 | Exa mpl e 7 | Exa mpl e 8 | Exa mpl e 9 | Exa mpl e 10 | Exa mpl e 11 | Exa mpl e 12 | Exa mple 13 |
| mNGF stock solution | Active ingredi ent (µg/mL ) | | | | | | | | 60 | | 100 | | | |
| hNGF stock solution | | | 40 | 40 | 80 | | 50 | 60 | | 50 | | 60 | 50 | 50 |
| rhNGF stock solution | | 10 | | | | 80 | | | | | | | | |
| glycine | Stabiliz er (mg/m L) | 1.5 | 3 | 6 | 3 | 4 | 3 | 9 | 9 | 5 | 3.5 | 9 | 2 | 5 |
| arginine | | 1.5 | 1 | 2 | 1.5 | 1.4 | 2 | 3 | 3 | 3 | 2 | 3 | 1.6 | 3 |
| glutami c acid | | 1.5 | 1.2 | 4 | 3 | 1.4 | 2.5 | 4 | 9 | 3.5 | 5.5 | 4 | 5.2 | 3.3 |
| isoleuci ne | | 1.5 | 1.3 | 2 | 5.5 | 2.1 | 2.5 | 4 | 9 | 3.5 | 3 | 4 | 4.2 | 4 |
| F68 | surfacta nt (mg/m L) | 1.0 | | | | | | | | | | | | |
| TW-20 | | | | | | | | | | | | | | 0.2 |
| TW-80 | | | | | | | | 0.2 | | | | | | |
| HS 15 | | | | | | | | | | | | | 0.5 | |
| mannito l | support ing agent (mg/m L) | 50 | 50 | 50 | 50 | 50 | | 40 | | 30 | 50 | 40 | 50 | 50 |
| dextrin | | | | | | | | | 20 | | | | | |
| Sorbitol | | | | | | | 40 | | | | | | | |
| phospha te | buffere d salt (mM) | | 25 | | 25 | | | | | | 10 | | | 20 |
| acetate | | | | | | 50 | | 20 | | | | 20 | | |
| citrate | | | | 25 | | | 25 | | | | | | 20 | |
| histidin e hydroch | | | | | | | | | 15 | 15 | | | | |
| loride | | | | | | | | | | | | | | |
| arginine hydroch loride | | 30 | | | | | | | 15 | 10 | | | | |
| pH in Exampl es | | 6.8 5 | 6.83 | 6.80 | 6.91 | 6.82 | 6.82 | 6.81 | 6.85 | 6.83 | 7.00 | 6.83 | 6.85 | 6.82 |

### Example 14. Performance test of the NGF compositions in the form of powder for injection prepared in Examples 1-13

The reference preparation in the following performance test uses the albumin-removing nerve growth factor lyophilized powder preparation in Example 14 in the patent with the Publication No. CN1318087C entitled "Albumin-removing Nerve Growth Factor Preparation".

### (1) Test of appearance, moisture, osmotic pressure, pH, content and activity

The NGF compositions in the form of powder for injection prepared in Examples 1-13 and a reference preparation are observed for the appearances, respectively, and their moistures, osmotic pressures, pHs, contents and activities are determined. The determination of the NGF content is carried out according to the method described in Example 1 in Patent Application No. 200510130348.3, entitled "Method for Determining the Content of Nerve Growth Factor". The activity test method is determined by a cell method, and the detailed method of operation is carried out according to the method of Example 1 in Patent Publication No. CN 103376248A, entitled "Method for Quantitative Determination of Nerve Growth Factor Activity". The experimental results are shown in Table 2.

**Table 2 Test results of test items in the Examples**

| Sample | Test results of the test items | | | | | |
|---|---|---|---|---|---|---|
| | Appearance | Moisture (%) | Osmotic pressure (mOsm/kg) | pH | Average content (%) | Average activity (U/bottle) |
| Reference preparation | white loose solid blocks | 1.1 | 245 | 7.03 | 99.87 | 12513 |
| Example 1 | white loose solid blocks | 1.7 | 149 | 6.95 | 98.38 | 8622 |
| Example 2 | white loose solid blocks | 1.6 | 152 | 7.02 | 99.16 | 19303 |
| Example 3 | white loose solid blocks | 1.1 | 157 | 6.98 | 99.23 | 18875 |
| Example 4 | white loose solid | 1.1 | 156 | 6.98 | 99.19 | 30706 |
| | blocks | | | | | |
| Example 5 | white loose solid blocks | 1.9 | 162 | 6.96 | 99.41 | 31926 |
| Example 6 | white loose solid blocks | 1.3 | 160 | 7.05 | 98.99 | 20930 |
| Example 7 | white loose solid blocks | 1.3 | 161 | 6.98 | 99.18 | 26777 |
| Example 8 | white loose solid blocks | 1.5 | 159 | 6.96 | 98.78 | 24011 |
| Example 9 | white loose solid blocks | 1.5 | 157 | 6.96 | 98.70 | 20121 |
| Example 10 | white loose solid blocks | 1.7 | 153 | 7.02 | 99.02 | 38136 |
| Example 11 | white loose solid blocks | 1.5 | 163 | 6.95 | 99.45 | 26338 |
| Example 12 | white loose solid blocks | 1.8 | 158 | 7.01 | 99.52 | 20878 |
| Example 13 | white loose solid blocks | 1.9 | 155 | 6.99 | 99.46 | 20612 |

Since the osmotic pressure is related to the ion concentration in the composition, the osmotic pressure varies in each of the above Examples, and the concentration of each Example in the present invention is slightly lower than the isotonic concentration, but does not cause significant discomfort to the patient. As can be seen from the results in the above table, the remaining indexes of the NGF compositions in the form of powder for injection prepared in the present invention all meet the quality standard requirements.

### (2) Stable pH range screening

According to the formulation composition of Example 3, 3 NGF compositions are formulated, wherein the total amount of citrate is kept constant and the relative ratio of citric acid to sodium citrate is adjusted so that the pH values of the NGF compositions are 6.0, 6.8 and 7.4, respectively, then filled into penicillin bottles, respectively, and lyophilized to prepare sterile powder for injection. Samples are taken, and an appropriate amount of sterile water for injection is added to formulate into solutions. Under room temperature condition, the solutions are observed for the appearance and determined for the pH value, the content and the activity at 0, 4, 8, 12 and 24 hours, respectively. The results are shown in the following table.

**Table 3. pH range screening of NGF compositions**

| Sample name | Storage time (h) | Appearance | pH | Average content (%) | Average activity (%) |
|---|---|---|---|---|---|
| Sample 1 (pH 6.0) | 0 | Colorless clear solution | 6.05 | 98.77 | 19553 |
| | 4 | Colorless clear solution | 5.99 | 99.02 | 20555 |
| | 8 | Colorless clear solution | 6.02 | 98.56 | 19393 |
| | 12 | Colorless clear solution | 6.01 | 98.11 | 19121 |
| | 24 | Colorless clear solution | 6.03 | 97.69 | 18826 |
| Sample 2 (pH 6.8) | 0 | Colorless clear solution | 6.79 | 98.68 | 19889 |
| | 4 | Colorless clear solution | 6.78 | 98.39 | 19701 |
| | 8 | Colorless clear solution | 6.80 | 98.18 | 20002 |
| | 12 | Colorless clear solution | 6.85 | 98.25 | 19535 |
| | 24 | Colorless clear solution | 6.84 | 98.01 | 19421 |
| Sample 3 (pH 7.4) | 0 | Colorless clear solution | 7.44 | 99.33 | 19312 |
| | 4 | Colorless clear solution | 7.39 | 99.46 | 19815 |
| | 8 | Colorless clear solution | 7.41 | 99.11 | 19111 |
| | 12 | Colorless clear solution | 7.41 | 98.89 | 18935 |
| | 24 | Colorless clear solution | 7.42 | 98.25 | 18658 |

It can be seen from the above table data that after the three NGF compositions with different pH values are stored at room temperature for 24 h, the indexes all have no significant changes than at 0 h, all meet the quality standard requirements, indicating that under room temperature condition, the sample solutions can remain stable within 24 h in a range of pH 6.0-7.4.

### (3) Accelerated Stability Test

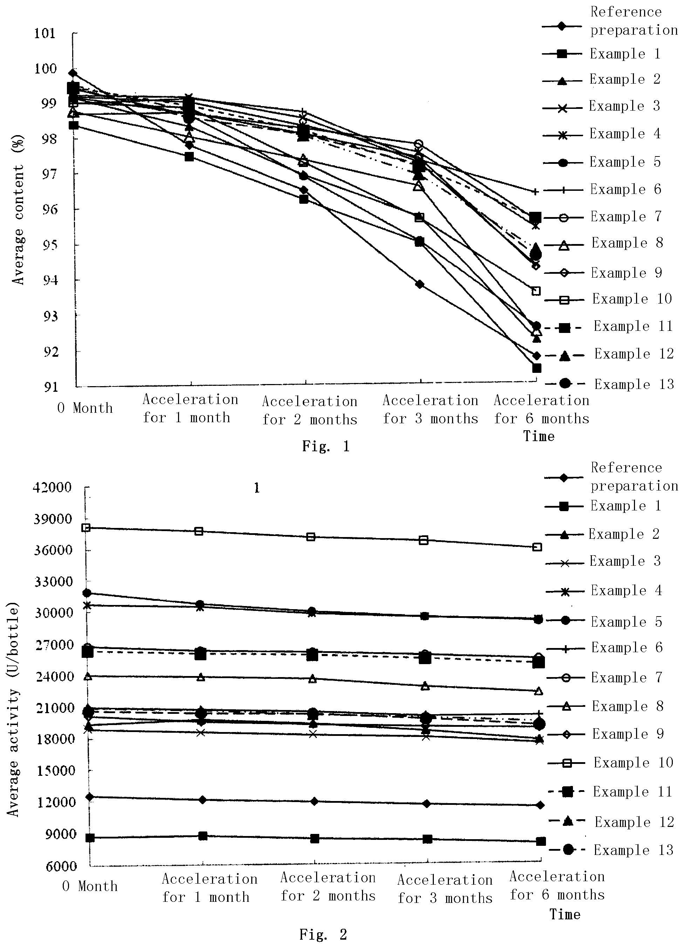

According to the requirements of "Technical Guidelines for Research on Stability of Biological Products", the samples of the reference preparation and the NGF compositions in the form of powder for injection prepared in the Examples 1-13 are stored under conditions of 25°C, RH 60 ± 10% for an accelerated stability examination for 0-6 months. The samples are observed for the appearances, and determined for the moistures, osmotic pressures, pH values, contents and activities. The results are shown in Table 4 and Fig. 1 (the change curve of average content over time), and Fig. 2 (the change curve of average activity over time, activity unit: U/bottle, volume per bottle: 0.63 ± 0.03 mL).

**Table 4. Accelerated stability test results of the NGF compositions in the form of powder for injection and the reference preparation**

| Time (month) | Sample | Appearance | Moisture (%) | Osmotic pressure (mOsm/kg) | pH | Average content (%) | Average activity (U/bottle) |
|---|---|---|---|---|---|---|---|
| 0 | Reference preparation | white loose solid blocks | 1.1 | 245 | 7.03 | 99.87 | 12513 |
| | Example 1 | white loose solid blocks | 1.7 | 149 | 6.95 | 98.38 | 8622 |
| | Example 2 | white loose solid blocks | 1.6 | 152 | 7.02 | 99.16 | 19303 |
| | Example 3 | white loose solid blocks | 1.1 | 157 | 6.98 | 99.23 | 18875 |
| | Example 4 | white loose solid blocks | 1.1 | 156 | 6.98 | 99.19 | 30706 |
| | Example 5 | white loose solid blocks | 1.9 | 162 | 6.96 | 99.41 | 31926 |
| | Example 6 | white loose solid blocks | 1.3 | 160 | 7.05 | 98.99 | 20930 |
| | Example 7 | white loose solid blocks | 1.3 | 161 | 6.98 | 99.18 | 26777 |
| | Example 8 | white loose solid blocks | 1.5 | 159 | 6.96 | 98.78 | 24011 |
| | Example 9 | white loose solid blocks | 1.5 | 157 | 6.96 | 98.70 | 20121 |
| | Example 10 | white loose solid blocks | 1.7 | 153 | 7.02 | 99.02 | 38136 |
| | Example 11 | white loose solid blocks | 1.5 | 163 | 6.95 | 99.45 | 26338 |
| | Example 12 | white loose solid blocks | 1.8 | 158 | 7.01 | 99.52 | 20878 |
| | Example 13 | white loose solid blocks | 1.9 | 155 | 6.99 | 99.46 | 20612 |
| 1 | Reference preparation | white loose solid blocks | 1.0 | 243 | 7.05 | 97.78 | 12116 |
| | Example 1 | white loose solid blocks | 1.8 | 152 | 6.96 | 97.46 | 8709 |
| | Example 2 | white loose solid blocks | 1.6 | 157 | 7.03 | 98.32 | 19712 |
| | Example 3 | white loose solid blocks | 1.1 | 159 | 6.97 | 99.15 | 18521 |
| | Example 4 | white loose solid blocks | 1.0 | 155 | 6.99 | 99.01 | 30425 |
| | Example 5 | white loose solid blocks | 1.8 | 163 | 6.96 | 98.65 | 30725 |
| | Example 6 | white loose solid blocks | 1.2 | 153 | 7.06 | 99.11 | 20655 |
| | Example 7 | white loose solid blocks | 1.2 | 159 | 6.99 | 98.70 | 26318 |
| | Example 8 | white loose solid blocks | 1.4 | 160 | 6.97 | 98.02 | 23825 |
| | Example 9 | white loose solid blocks | 1.3 | 157 | 6.96 | 98.71 | 19505 |
| | Example 10 | white loose solid blocks | 1.8 | 155 | 7.03 | 98.85 | 37687 |
| | Example 11 | white loose solid blocks | 1.4 | 161 | 6.95 | 98.91 | 26013 |
| | Example 12 | white loose solid blocks | 1.7 | 160 | 7.03 | 98.78 | 20602 |
| | Example 13 | white loose solid blocks | 1.9 | 157 | 6.96 | 98.58 | 20355 |
| | Reference | white loose | 0.9 | 249 | 7.05 | 96.48 | 11856 |
| 2 | preparation | solid blocks | | | | | |
| | Example 1 | white loose solid blocks | 1.8 | 154 | 6.97 | 96.22 | 8328 |
| | Example 2 | white loose solid blocks | 1.7 | 162 | 7.03 | 96.92 | 19265 |
| | Example 3 | white loose solid blocks | 1.0 | 155 | 6.97 | 98.55 | 18221 |
| | Example 4 | white loose solid blocks | 1.0 | 153 | 6.98 | 98.32 | 29705 |
| | Example 5 | white loose solid blocks | 1.7 | 162 | 6.97 | 96.88 | 29889 |
| | Example 6 | white loose solid blocks | 1.3 | 157 | 7.06 | 98.71 | 20382 |
| | Example 7 | white loose solid blocks | 1.2 | 160 | 6.97 | 98.25 | 26098 |
| | Example 8 | white loose solid blocks | 1.3 | 164 | 6.97 | 97.35 | 23528 |
| | Example 9 | white loose solid blocks | 1.2 | 160 | 6.98 | 98.09 | 19201 |
| | Example 10 | white loose solid blocks | 1.7 | 158 | 7.03 | 97.26 | 36998 |
| | Example 11 | white loose solid blocks | 1.5 | 160 | 6.98 | 98.19 | 25798 |
| | Example 12 | white loose solid blocks | 1.6 | 155 | 7.02 | 98.02 | 20135 |
| | Example 13 | white loose solid blocks | 1.9 | 158 | 7.02 | 98.07 | 20172 |
| 3 | Reference preparation | white loose solid blocks | 1.0 | 241 | 7.04 | 93.77 | 11511 |
| | Example 1 | white loose solid blocks | 1.8 | 150 | 6.98 | 94.98 | 8185 |
| | Example 2 | white loose solid | 1.3 | 150 | 7.03 | 95.73 | 18535 |
| | | blocks | | | | | |
| | Example 3 | white loose solid blocks | 1.1 | 158 | 6.99 | 97.25 | 17921 |
| | Example 4 | white loose solid blocks | 1.0 | 157 | 6.97 | 97.53 | 29333 |
| | Example 5 | white loose solid blocks | 1.8 | 160 | 6.95 | 95.03 | 29323 |
| | Example 6 | white loose solid blocks | 1.1 | 162 | 7.06 | 97.31 | 19933 |
| | Example 7 | white loose solid blocks | 1.4 | 160 | 6.99 | 97.76 | 25787 |
| | Example 8 | white loose solid blocks | 1.4 | 160 | 6.98 | 96.58 | 22722 |
| | Example 9 | white loose solid blocks | 1.3 | 156 | 6.97 | 97.39 | 18922 |
| | Example 10 | white loose solid blocks | 1.8 | 155 | 7.03 | 95.68 | 36585 |
| | Example 11 | white loose solid blocks | 1.5 | 160 | 6.96 | 97.13 | 25413 |
| | Example 12 | white loose solid blocks | 1.9 | 157 | 7.03 | 96.89 | 19821 |
| | Example 13 | white loose solid blocks | 1.8 | 155 | 7.00 | 97.19 | 19651 |
| 6 | Reference preparation | white loose solid blocks | 0.9 | 242 | 7.05 | 91.73 | 11237 |
| | Example 1 | white loose solid blocks | 1.7 | 150 | 6.98 | 91.39 | 7819 |
| | Example 2 | white loose solid blocks | 1.5 | 153 | 7.03 | 92.23 | 17617 |
| | Example 3 | white loose solid blocks | 0.9 | 156 | 6.97 | 94.32 | 17352 |
| | Example 4 | white loose solid blocks | 1.1 | 155 | 6.96 | 95.41 | 28939 |
| | Example 5 | white loose solid blocks | 1.8 | 164 | 6.98 | 92.58 | 28887 |
| | Example 6 | white loose solid blocks | 1.3 | 162 | 7.07 | 96.36 | 19921 |
| | Example 7 | white loose solid blocks | 1.4 | 163 | 6.99 | 95.61 | 25323 |
| | Example 8 | white loose solid blocks | 1.1 | 160 | 6.98 | 92.45 | 22128 |
| | Example 9 | white loose solid blocks | 1.0 | 159 | 6.97 | 94.26 | 18711 |
| | Example 10 | white loose solid blocks | 1.6 | 155 | 7.04 | 93.55 | 35825 |
| | Example 11 | white loose solid blocks | 1.4 | 158 | 6.97 | 95.65 | 24873 |
| | Example 12 | white loose solid blocks | 1.8 | 159 | 7.03 | 94.76 | 19321 |
| | Example 13 | white loose solid blocks | 1.8 | 151 | 6.97 | 94.58 | 18999 |

As can be seen from Fig. 1, the average content of NGF in the reference preparation and the NGF compositions in the form of powder for injection prepared in the Examples presents a reduction tendency over time under accelerated conditions (25°C, RH 60 ± 10%), wherein the reduction speed in the average content of NGF in the reference preparation is greater than that of Examples of the present invention. As can be seen from the results in Table 4, the appearances, moistures, pH values and osmotic pressures of the samples are not significantly changed under the accelerated conditions (25°C, RH 60 ± 10%) for 6 months, but the content of the reference preparation reduces by about 8.2%, the reduction rates in the contents of Examples 1 to 13 are 7.1%, 7.0%, 4.9%, 3.8%, 6.9%, 2.7%, 3.6%, 6.4%, 4.5%, 5.5%, 3.8%, 4.8% and 4.9%, respectively. Therefore, the stability of the Examples 1-13 is superior to the reference preparation under accelerated conditions, wherein the stability of Examples 3, 4, 6, 7 and 11, particularly Examples 4, 6, 7 and 11 is significantly superior to that of the reference preparation.

As can be seen from Fig. 2, the average activity of NGF in the reference preparation and the NGF compositions in the form of powder for injection prepared in the Examples presents a reduction tendency over time under accelerated conditions (25°C, RH 60 ± 10%), but as can be seen from Fig. 2, the reduction speed in the average activity of the reference preparation is greater than that of the NGF compositions in the form of powder for injection prepared in the present invention. As can be seen from the results in Table 4, the appearances, moistures, pH values and osmotic pressures of the samples are not significantly changed under accelerated conditions (25°C, RH 60 ± 10%) for 6 months, the activity of the reference preparation reduces by 10.2%, Examples 1 to 13 reduce by 9.3%, 8.7%, 8.1%, 5.8%, 9.5%, 4.8%, 5.4%, 7.8%, 7.0%, 6.1%, 5.6%, 7.5% and 7.8%, respectively. Therefore, the stability of the Examples 1-13 is superior to the reference preparation under accelerated conditions, wherein the stability of the Examples 4, 6, 7 and 11, particularly Examples 4, 6, 7 and 11 is significantly superior to that of the reference preparation.

### B. Long-term Stability Test

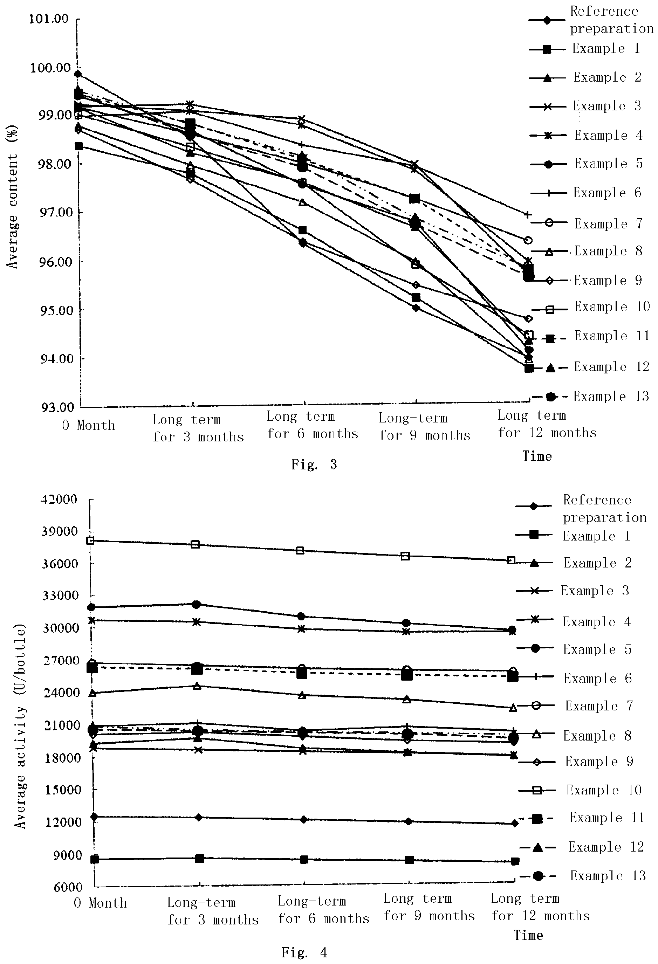

According to the requirements of "Technical Guidelines for Research on Stability of Biological Products", the samples of the reference preparation and the NGF powders for injection prepared in the Examples 1-13 are examined for a long-term stability for 0-12 months under condition of 6 ± 2°C. The samples are observed for the appearances, and determined for the moistures, osmotic pressures, pH values, contents and activities. The results are shown in Table 5, and Fig. 3 (the change curve of average content over time) and Fig. 4 (the change curve of average activity over time, activity unit: U/bottle, volume per bottle: 0.63 ± 0.03 mL).

**Table 5. Long-term stability test results of the NGF compositions in the form of powder for injection and the reference preparation**

| time (month) | Sample | Appearance | Moisture (%) | Osmotic pressure (mOsm/kg) | pH | Average content (%) | Average activity (U/bottle) |
|---|---|---|---|---|---|---|---|
| 0 | Reference preparation | white loose solid blocks | 1.1 | 245 | 7.03 | 99.87 | 12513 |
| | Example 1 | white loose solid blocks | 1.7 | 149 | 6.95 | 98.38 | 8622 |
| | Example 2 | white loose solid blocks | 1.6 | 152 | 7.02 | 99.16 | 19303 |
| | Example 3 | white loose solid blocks | 1.1 | 157 | 6.98 | 99.23 | 18875 |
| | Example 4 | white loose solid blocks | 1.1 | 156 | 6.98 | 99.19 | 30706 |
| | Example 5 | white loose solid blocks | 1.9 | 162 | 6.96 | 99.41 | 31926 |
| | Example 6 | white loose solid blocks | 1.3 | 160 | 7.05 | 98.99 | 20930 |
| | Example 7 | white loose solid blocks | 1.3 | 161 | 6.98 | 99.18 | 26777 |
| | Example 8 | white loose solid blocks | 1.5 | 159 | 6.96 | 98.78 | 24011 |
| | Example 9 | white loose solid blocks | 1.5 | 157 | 6.96 | 98.70 | 20121 |
| | Example 10 | white loose solid blocks | 1.7 | 153 | 7.02 | 99.02 | 38136 |
| | Example 11 | white loose solid blocks | 1.5 | 163 | 6.95 | 99.45 | 26338 |
| | Example 12 | white loose solid blocks | 1.8 | 158 | 7.01 | 99.52 | 20878 |
| | Example 13 | white loose solid blocks | 1.9 | 155 | 6.99 | 99.46 | 20612 |
| 3 | Reference preparation | white loose solid blocks | 1.2 | 244 | 7.03 | 98.53 | 12358 |
| | Example 1 | white loose solid blocks | 1.6 | 151 | 6.97 | 97.78 | 8583 |
| | Example 2 | white loose solid blocks | 1.5 | 150 | 7.05 | 98.21 | 19719 |
| | Example 3 | white loose solid blocks | 1.0 | 155 | 7.00 | 99.08 | 18623 |
| | Example 4 | white loose solid blocks | 1.0 | 155 | 6.99 | 99.21 | 30452 |
| | Example 5 | white loose solid blocks | 1.8 | 161 | 6.97 | 98.67 | 32087 |
| | Example 6 | white loose solid blocks | 1.3 | 162 | 7.05 | 99.07 | 21109 |
| | Example 7 | white loose solid blocks | 1.4 | 160 | 6.99 | 98.61 | 26426 |
| | Example 8 | white loose solid blocks | 1.4 | 157 | 6.97 | 97.95 | 24557 |
| | Example 9 | white loose solid blocks | 1.5 | 155 | 6.95 | 97.65 | 20230 |
| | Example 10 | white loose solid blocks | 1.6 | 154 | 7.04 | 98.33 | 37658 |
| | Example 11 | white loose solid blocks | 1.5 | 160 | 6.94 | 98.82 | 26103 |
| | Example 12 | white loose solid blocks | 1.7 | 159 | 7.00 | 98.81 | 20512 |
| | Example 13 | white loose solid blocks | 1.7 | 155 | 6.99 | 98.61 | 20401 |
| 6 | Reference preparation | white loose solid blocks | 0.9 | 243 | 7.03 | 96.31 | 12038 |
| | Example 1 | white loose solid blocks | 1.6 | 152 | 6.97 | 96.59 | 8339 |
| | Example 2 | white loose solid blocks | 1.6 | 150 | 7.03 | 97.57 | 18693 |
| | Example 3 | white loose solid blocks | 1.2 | 158 | 6.97 | 98.89 | 18395 |
| | Example 4 | white loose solid blocks | 0.9 | 155 | 6.98 | 98.76 | 29705 |
| | Example 5 | white loose solid blocks | 1.9 | 164 | 6.96 | 97.52 | 30825 |
| | Example 6 | white loose solid blocks | 1.1 | 159 | 7.06 | 98.35 | 20318 |
| | Example 7 | white loose solid blocks | 1.3 | 163 | 6.97 | 97.98 | 26078 |
| | Example 8 | white loose solid blocks | 1.5 | 161 | 6.97 | 97.17 | 23578 |
| | Example 9 | white loose solid blocks | 1.3 | 155 | 6.96 | 96.35 | 19771 |
| | Example 10 | white loose solid blocks | 1.6 | 155 | 7.04 | 97.56 | 37015 |
| | Example 11 | white loose solid blocks | 1.6 | 161 | 6.97 | 98.05 | 25631 |
| | Example 12 | white loose solid blocks | 1.7 | 157 | 7.03 | 98.12 | 20221 |
| | Example 13 | white loose solid blocks | 1.9 | 158 | 7.02 | 97.89 | 20112 |
| 9 | Reference preparation | white loose solid blocks | 1.0 | 242 | 7.04 | 94.96 | 11758 |
| | Example 1 | white loose solid blocks | 1.6 | 152 | 6.97 | 95.17 | 8172 |
| | Example 2 | white loose solid blocks | 1.3 | 149 | 7.05 | 96.63 | 18198 |
| | Example 3 | white loose solid blocks | 1.0 | 156 | 6.96 | 97.93 | 18113 |
| | Example 4 | white loose solid blocks | 1.0 | 158 | 6.95 | 97.81 | 29333 |
| | Example 5 | white loose solid blocks | 1.8 | 160 | 6.97 | 96.75 | 30128 |
| | Example 6 | white loose solid blocks | 1.2 | 162 | 7.05 | 97.89 | 20582 |
| | Example 7 | white loose solid blocks | 1.4 | 162 | 6.98 | 97.22 | 25791 |
| | Example 8 | white loose solid blocks | 1.3 | 158 | 6.97 | 95.92 | 23065 |
| | Example 9 | white loose solid blocks | 1.4 | 157 | 6.96 | 95.43 | 19309 |
| | Example 10 | white loose solid blocks | 1.7 | 155 | 7.04 | 95.85 | 36365 |
| | Example 11 | white loose solid blocks | 1.6 | 159 | 6.93 | 97.21 | 25328 |
| | Example 12 | white loose solid blocks | 1.9 | 155 | 7.03 | 96.82 | 19989 |
| | Example 13 | white loose solid blocks | 1.9 | 152 | 6.96 | 96.69 | 19885 |
| 12 | Reference preparation | white loose solid blocks | 1.0 | 242 | 7.02 | 93.93 | 11462 |
| | Example 1 | white loose solid blocks | 1.8 | 146 | 6.96 | 93.69 | 7958 |
| | Example 2 | white loose solid blocks | 1.5 | 153 | 7.04 | 94.26 | 17796 |
| | Example 3 | white loose solid blocks | 1.0 | 155 | 6.95 | 95.63 | 17858 |
| | Example 4 | white loose solid blocks | 1.0 | 157 | 6.98 | 95.91 | 29239 |
| | Example 5 | white loose solid blocks | 1.8 | 161 | 6.97 | 94.08 | 29388 |
| | Example 6 | white loose solid blocks | 1.1 | 162 | 7.05 | 96.85 | 20078 |
| | Example 7 | white loose solid blocks | 1.2 | 162 | 6.97 | 96.33 | 25585 |
| | Example 8 | white loose solid blocks | 1.6 | 160 | 6.97 | 93.88 | 22172 |
| | Example 9 | white loose solid blocks | 1.5 | 158 | 6.98 | 94.71 | 19011 |
| | Example 10 | white loose solid blocks | 1.7 | 154 | 7.03 | 94.38 | 35869 |
| | Example 11 | white loose solid blocks | 1.5 | 165 | 6.98 | 95.73 | 25065 |
| | Example 12 | white loose solid blocks | 1.7 | 159 | 7.03 | 95.76 | 19712 |
| | Example 13 | white loose solid blocks | 1.8 | 158 | 7.02 | 95.58 | 19409 |

As can be seen from Fig. 3, the average content of NGF in the reference preparation and the NGF compositions in the form of powder for injection prepared in the Examples presents a reduction tendency over time under the long-term stability condition (6 ± 2°C), wherein the reduction speed in the average content of NGF in the reference preparation is greater than that of Examples of the present invention. As can be seen from the results in Table 5, the appearances, moistures, pH values and osmotic pressures of the samples are not significantly changed under the long-term condition (6 ± 2°C) for 12 months, the content of the reference preparation reduces by about 5.9%, the reduction rates in the contents of Examples 1 to 13 are 4.8%, 4.9%, 3.6%, 3.3%, 5.4%, 2.2%, 2.9%, 5.0%, 4.0%, 4.7%, 3.7%, 3.8% and 3.9%, respectively. Therefore, the stability of the Examples 1-13 is superior to the reference preparation under the long-term condition, wherein the stability of Examples 3, 4, 6, 7 and 11, particularly Examples 4, 6, 7 and 11 is significantly superior to that of the reference preparation.

As can be seen from Fig. 4, the average activity of NGF in the reference preparation and the NGF compositions in the form of powder for injection prepared in the Examples presents a reduction tendency over time as a whole under the long-term stability condition (6 ± 2°C), however, as can be seen from Fig. 4, the reduction speed in the activity of the reference preparation is greater than that of Examples of the present invention. As can be seen from the results in Table 5, the appearances, moistures, pH values and osmotic pressures of the samples are not significantly changed under the long-term condition for 12 months, the activity of the reference preparation reduces by 8.4%, the activities of Examples 1-13 reduce by 7.7%, 7.8%, 5.4%, 4.8%, 7.9%, 4.1%, 4.5%, 7.7%, 5.5%, 5.9%, 4.8%, 5.6% and 5.8% respectively. Therefore, the stability of the Examples 1-13 is superior to the reference preparation under the long-term condition, wherein the stability of Examples 3, 4, 6, 7 and 11, particularly Examples 4, 6, 7 and 11 is significantly superior to that of the reference preparation.

As can be seen from the above stability test results, compared with the reference preparation using alanine, arginine and glycine as a stabilizer, the NGF compositions in the form of powder for injection of the present invention, particularly Examples 4, 6, 7 and 11, using the four amino acids of arginine, glutamic acid, glycine and isoleucine as a stabilizer, have a more excellent stability.

### (4) Clinical evaluation

A total of 271 patients with optic nerve injury are subjected to a 12-weeks clinical trial by the multicenter non-randomized controlled clinical study design; all the subjects are 14 years or older, and are able to communicate well with the investigators, understand and comply with clinical trial requirements, and sign an informed consent.

Subject condition: 14 years or older.

Gender: male or female.

Subject source: 409 patients with optic nerve injury caused by various causes from various ophthalmic research units being enrolled.

Injury cause: 136 cases of fist injury (136 eyes), 152 cases of car accident (152 eyes), 73 cases of physical hit injury (73 eyes), 49 cases of eye explosive injury (49 eyes). All cases are not accompanied by eyeball rupture and damage caused by the optic nerve compression due to orbital fractures.

Treatment method: the treatment group is injected with the reference preparation and the NGF compositions of the present invention by intramuscular injection once a day with each 30 µg, and all cases are administered continuously for 12 weeks. The placebo group is treated with a negative control (with no active ingredient, and the remaining excipients being the same to those in the NGF compositions).

The treatment group comprises 271 cases (271 eyes), with age of 18 to 63 years, and the average age of 34.2 years; 189 males (189 eyes), 82 females (82 eyes); 147 eyes in the right eye, 124 eyes in the left eye. The placebo group comprises 138 patients (138 eyes), with age of 13-55 years, and the average age of 33.9 years, 96 males (96 eyes), 42 females (42 eyes), 45 eyes in the right eye and 93 eyes in the left eye.

Grouping design: grouping is shown in the following table:

**Table 6 Grouping of clinical trial and number of subjects**

| Test group | Test grouping and number of subjects (person) | | |
|---|---|---|---|
| | Placebo group | Reference preparation group | NGF composition group |
| Fist injury | 46 | 45 | 45 |
| Car accident | 50 | 51 | 51 |
| Physical hit injury | 25 | 24 | 24 |
| Explosive injury | 17 | 16 | 16 |
| Number of subjects in each group | 138 | 135 | 136 |

| | | | |
|---|---|---|---|
| Note: in the NGF composition group of the above table, the nerve growth factor composition prepared in Example 6 is used, the same below. | | | |

Clinical medicine efficacy: patients are administrated with the placebo, the reference preparation and the NGF composition in the form of powder for injection by intramuscular injection, and the therapeutic effect is evaluated according to the recovery, effective, alleviated and ineffective etc. The results are as follows:

**Table 7 Comparison of NGF clinical efficacy**

| Clinical efficacy | Placebo group (N = 138) | Reference preparation group (N = 135) | NGF composition group (N = 136) |
|---|---|---|---|
| Recovery | 3 (2.2) | 82 (60.7) | 96 (70.6) |
| Effective | 11 (8.0) | 24 (17.8) | 29 (21.3) |
| Alleviated | 9 (6.5) | 19 (14.1) | 10 (7.4) |
| Ineffective | 115 (83.3) | 10 (7.4) | 1 (0.7) |

The clinical efficacy in the above table is evaluated using the following clinical efficacy evaluation criteria:
① Recovery: the vision recovers to 1.0 or more, and dark spots in the central visual field disappear;
② Effective: the vision increases by 4 lines or more, dark spots in the central visual field reduce or the absolute dark spots become relative dark spots;
③ Alleviated: the vision increases by 2 lines or more, and there is no change in visual field;
④ Ineffective: the vision and visual field are the same as before the treatment, or decline.

It can be seen from the above table that, compared with the placebo group, the NGF compositions of the present invention can be effective in treating optic nerve injury caused by various causes, have the efficiency of the treatment of optic nerve injury that is superior to that of the reference preparation group, and have good clinical therapeutic effect.

### Industrial application

The nerve growth factor compositions and the powders for injection prepared in the present invention retain good stability in preparation, transportation and storage processes: (1) in the preparation process, after the nerve growth factor compositions in the form of powder for injection prepared in the present invention are placed at room temperature for 24 hours, the content and activity of the NGF therein have no significant change; (2) compared with the reference preparation, in the conventional (6 ± 2°C) transportation and storage processes, the NGF composition in the form of powder for injection of the present invention has an effective content reduction of only 2.4%-3.4% and an small activity reduction, after being placed for 12 months, thereby having the excellent stability.

The nerve growth factor compositions in the form of powder for injection prepared in the present invention can significantly reduce the incidence of the adverse reactions in the clinical trial, have good clinical therapeutic effect, and have better clinical medication safety and quality controllability as compared with the existing reference preparation.

The present invention has the following beneficial effects:
(1) the nerve growth factor compositions and the powders for injection of the present invention can avoid the potential risk resulting from the virus or other unknown components carried in albumin by using an amino acid instead of albumin as a stabilizer.
(2) the nerve growth factor compositions in the form of powder for injection of the present invention not only have protective effect on mouse nerve growth factor (mNGF), but also can ensure the good stability of the human nerve growth factor (hNGF) and the recombinant human nerve growth factor (rhNGF) in the preparation, transportation and storage processes, and have better clinical medication safety and quality controllability.
(3) in the nerve growth factor compositions and the powders for injection of the present invention, the compositions have definite ingredients, are easily qualitative and quantitative, and the stabilizer used therein has a high purity, a wide source and easy long-term mass production, which facilitate the cost control and the product quality improvement.
(4) The nerve growth factor compositions in the form of powders for injection of the present invention can solve the stability problem of the product long-term storage very well and has a high clinical effectiveness.

## Claims

1. A nerve growth factor composition, **characterized in that** said nerve growth factor composition comprises a nerve growth factor, a stabilizer, a supporting agent, a pH buffer and a water;
said stabilizer is arginine, glutamic acid, glycine and isoleucine and
said supporting agent is any one of mannitol, dextrin and sorbitol.

2. The nerve growth factor composition according to claim 1, **characterized in that** in said nerve growth factor composition:
a mass-volume concentration of said nerve growth factor is 10 µg/mL-100 (µg/mL;
a mass-volume concentration of said stabilizer is 6 mg/mL-30 mg/mL;
a mass-volume concentration of said supporting agent is 20 mg/mL-50 mg/mL;
said pH buffer maintains said nerve growth factor composition at a pH value of 6.80 to 7.00;
solvent is water.

3. The nerve growth factor composition according to claim 1 or 2, **characterized in that** the mass-volume concentration of said stabilizer is 10 mg/mL-20 mg/mL.

4. The nerve growth factor composition according to any one of claims 1 to 3, **characterized in that** the mass ratio of arginine to glutamic acid to glycine to isoleucine in said stabilizer is 1 : (1-3) : (1-3) : (1-3.6), preferably, the mass ratio of arginine to glutamic acid to glycine to isoleucine in said stabilizer is 1 : 1.25 : 1.5 : 1.25.

5. The nerve growth factor composition according to any one of claims 1 to 4, **characterized in that** a mole concentration of said pH buffer is 10 mM-50 mM, preferably 20 mM-25 mM.

6. The nerve growth factor composition according to any one of claims 1 to 5, **characterized in that** said pH buffer is selected from one or more of a phosphate, a citrate, an acetate, a histidine hydrochloride and an arginine hydrochloride.

7. The nerve growth factor composition according to any one of claims 1 to 6, **characterized in that** the mass-volume concentration of said nerve growth factor is 40 µg/mL-80 µg/mL,

8. The nerve growth factor composition according to any one of claims 1 to 9, **characterized in that** in said nerve growth factor composition further comprises a surfactant.

9. The nerve growth factor composition according to any one of claims 1 to 8, **characterized in that** a mass-volume concentration of said surfactant is 0 mg/mL-1.0 mg/mL, but not 0; preferably, the mass-volume concentration of said surfactant is 0.2 mg/mL-0.5 mg/mL.

10. The nerve growth factor composition according to any one of claims 1 to 9, **characterized in that** said surfactant is a nonionic surfactant, preferably said surfactant is at least one of poloxamer, polysorbate and 15-polyethleneglycol hydroxystearate.

11. The nerve growth factor composition according to any one of claims 1 to 10, **characterized in that** in said nerve growth factor composition:
the mass-volume concentration of said nerve growth factor is 50 µg/mL;
the mass-volume concentration of said stabilizer is 10 mg/mL;
the mass-volume concentration of said supporting agent is 40 mg/mL;
the mole concentration of said pH buffer is 25 mM.

12. The nerve growth factor composition according to any one of claims 1 to 11, **characterized in that** said nerve growth factor is selected from a mouse-derived nerve growth factor, a human-derived nerve growth factor, or a recombinant human nerve growth factor.

13. A method for preparation of a nerve growth factor composition in the form of powder for injection, **characterized in that** the nerve growth factor composition according to any one of claims 1 to 12 is lyophilized to obtain said nerve growth factor composition in the form of powder for injection.

14. A nerve growth factor composition in the form of powder for injection prepared by the method according to claim 13.

15. A nerve growth factor composition according to any one of claims 1 to 14 for use in the treatment of a nerve injury, wherein the nerve injury may be an optic nerve injury.

## Patentansprüche

1. Eine Nervenwachstumsfaktorzusammensetzung, **dadurch gekennzeichnet, dass** besagte Nervenwachstumsfaktorzusammensetzung einen Nervenwachstumsfaktor, einen Stabilisator, ein Unterstützungsmittel, einen pH Puffer und ein Wasser umfasst;
der besagte Stabilisator ist Arginin, Glutaminsäure, Glycin, und Isoleucin und
das besagte Unterstützungsmittel ist eines der folgenden Mannitol, Dextrin, und Sorbitol.

2. Die Nervenwachstumsfaktorzusammensetzung gemäß Anspruch 1, **dadurch gekennzeichnet, dass** in besagter Nervenwachstumsfaktorzusammensetzung:
eine Massen-Volumen-Konzentration des besagten Nervenwachstumsfaktors ist 10 µg/mL - 100 µg/mL;
eine Massen-Volumen-Konzentration des besagten Stabilisators ist 6 mg/mL - 30 mg/mL;
eine Massen-Volumen-Konzentration des besagten Unterstützungsmittels ist 20 mg/mL - 50 mg/mL;
besagter pH Puffer hält die besagte Nervenwachstumsfaktorzusammensetzung bei einem pH Wert von 6,80 bis 7,00;
Lösungsmittel ist Wasser.

3. Die Nervenwachstumsfaktorzusammensetzung gemäß Anspruch 1 oder 2, **dadurch gekennzeichnet, dass** die Massen-Volumen-Konzentration des besagten Stabilisators 10 mg/mL - 20 mg/mL ist.

4. Die Nervenwachstumsfaktorzusammensetzung gemäß einem der Ansprüche 1 bis 3, **dadurch gekennzeichnet, dass** das Massenverhältnis von Arginin zu Glutaminsäure zu Glycin zu Isoleucin in besagtem Stabilisator ist 1 : (1-3) : (1-3) : (1-3,6), vorzugsweise ist das Massenverhältnis von Arginin zu Glutaminsäure zu Glycin zu Isoleucin in besagtem Stabilisator 1 : 1,25 : 1,5 : 1,25.

5. Die Nervenwachstumsfaktorzusammensetzung gemäß einem der Ansprüche 1 bis 4, **dadurch gekennzeichnet, dass** die Molkonzentration des besagten pH Puffers ist 10 mM-50 mM, vorzugsweise 20 mM-25mM.

6. Die Nervenwachstumsfaktorzusammensetzung gemäß einem der Ansprüche 1 bis 5, **dadurch gekennzeichnet, dass** der pH Puffer ausgewählt ist aus einem oder mehreren aus einem Phosphat, einem Citrat, einem Acetat, einem Histidinhydrochlorid und einem Argininhydrochlorid.

7. Die Nervenwachstumsfaktorzusammensetzung gemäß einem der Ansprüche 1 bis 6, **dadurch gekennzeichnet, dass** die Massen-Volumen-Konzentration von besagtem Nervenwachstumsfaktor 40 µg/mL - 80 µg/mL ist.

8. Die Nervenwachstumsfaktorzusammensetzung gemäß einem der Ansprüche 1 bis 9, **dadurch gekennzeichnet, dass** die Nervenwachstumsfaktorzusammensetzung weiterhin ein Tensid umfasst.

9. Die Nervenwachstumsfaktorzusammensetzung gemäß einem der Ansprüche 1 bis 8, **dadurch gekennzeichnet, dass** die Massen-Volumen-Konzentration von besagtem Tensid ist 0 mg/ml - 1.0 mg/mL, aber nicht 0, vorzugsweise ist die Massen-Volumen-Konzentration von besagtem Tensid 0,2 mg/mL - 0,5 mg/mL.

10. Die Nervenwachstumsfaktorzusammensetzung gemäß einem der Ansprüche 1 bis 9, **dadurch gekennzeichnet, dass** besagtes Tensid ein nichtionisches Tensid ist, vorzugsweise ist das Tensid mindestens ein Poloxamer, Polysorbat und 15-Polyethylenglycolhydroxystearat.

11. Die Nervenwachstumsfaktorzusammensetzung gemäß einem der Ansprüche 1 bis 10, **dadurch gekennzeichnet, dass** in besagter Nervenwachstumsfaktorzusammensetzung:
die Massen-Volumen-Konzentration des Nervenwachstumsfaktors ist 50 µg/mL;
die Massen-Volumen-Konzentration des besagten Stabilisators ist 10 mg/mL;
die Massen-Volumen-Konzentration des besagten Unterstützungsmittels ist 40 mg/mL;
die Molkonzentration des besagten pH Puffers ist 25 mM.

12. Die Nervenwachstumsfaktorzusammensetzung gemäß einem der Ansprüche 1 bis 11, **dadurch gekennzeichnet, dass** besagter Nervenwachstumsfaktor ausgewählt ist aus einem von Mäusen abgeleiteten Nervenwachstumsfaktor, einem vom Menschen abgeleiteten Nervenwachstumsfaktor, oder einem rekombinanten menschlichen Nervenwachstumsfaktor.

13. Ein Verfahren zur Herstellung einer Nervenwachstumsfaktorzusammensetzung in der Form eines Pulvers zur Injektion, **dadurch gekennzeichnet, dass** die Nervenwachstumsfaktorzusammensetzung gemäß einem der Ansprüche 1-12 lyophilisiert wird um besagte Nervenwachstumsfaktorzusammensetzung in Form von Pulver zur Injektion zu erhalten.

14. Eine Nervenwachstumstumsfaktorzusammensetzung in der Form von Pulver für Injektion, hergestellt durch das Verfahren gemäß Anspruch 13.

15. Eine Nervenwachstumstumsfaktorzusammensetzung gemäß einem der Ansprüche 1 bis 14 zur Verwendung in der Behandlung einer Nervenverletzung, wobei die Nervenverletzung eine Verletzung des optischen Nervs sein kann.

## Revendications

1. Une composition de facteur de croissance nerveuse, **caractérisée en ce que** ladite composition de facteur de croissance nerveuse comprend un facteur de croissance nerveuse, un stabilisant, un agent de support, un tampon de pH et une eau ;
ledit stabilisant est l'arginine, l'acide glutamique, la glycine et l'isoleucine, et
ledit agent de support est l'un quelconque parmi le mannitol, la dextrine et le sorbitol.

2. La composition de facteur de croissance nerveuse selon la revendication 1, **caractérisée en ce que** dans ladite composition de facteur de croissance nerveuse :
une concentration masse-volume dudit facteur de croissance nerveuse va de 10 µg/ml à 100 µg/ml ;
une concentration masse-volume dudit stabilisant va de 6 mg/ml à 30 mg/ml ;
une concentration masse-volume dudit agent de support va de 20 mg/ml à 50 mg/ml ;
ledit tampon de pH maintient ladite composition de facteur de croissance nerveuse à une valeur de pH allant de 6,80 à 7,00 ;
le solvant est de l'eau.

3. La composition de facteur de croissance nerveuse selon la revendication 1 ou la revendication 2, **caractérisée en ce que** la concentration masse-volume dudit stabilisant est de 10 mg/ml à 20 mg/ml.

4. La composition de facteur de croissance nerveuse selon l'une quelconque des revendications 1 à 3, **caractérisée en ce que** le rapport massique de l'arginine à l'acide glutamique à la glycine à l'isoleucine dans ledit stabilisant est de 1 : (1-3) : (1-3) : (1-3,6), de préférence, le rapport massique de l'arginine à l'acide glutamique à la glycine à l'isoleucine dans ledit stabilisant est de 1 : 1,25 : 1,5 : 1,25.

5. La composition de facteur de croissance nerveuse selon l'une quelconque des revendications 1 à 4, **caractérisée en ce qu'**une concentration molaire dudit tampon de pH va de 10 mM à 50 mM, de préférence de 20 mM à 25 mM.

6. La composition de facteur de croissance nerveuse selon l'une quelconque des revendications 1 à 5, **caractérisée en ce que** ledit tampon de pH est choisi parmi un ou plusieurs parmi un phosphate, un citrate, un acétate, un chlorhydrate d'histidine et un chlorhydrate d'arginine.

7. La composition de facteur de croissance nerveuse selon l'une quelconque des revendications 1 à 6, **caractérisée en ce que** la concentration masse-volume dudit facteur de croissance nerveuse est de 40 µg/ml à 80 µg/ml.

8. La composition de facteur de croissance nerveuse selon l'une quelconque des revendications 1 à 9, **caractérisée en ce que** ladite composition de facteur de croissance nerveuse comprend en outre un tensioactif.

9. La composition de facteur de croissance nerveuse selon l'une quelconque des revendications 1 à 8, **caractérisée en ce qu'**une concentration masse-volume dudit tensioactif va de 0 mg/ml à 1,0 mg/ml, mais n'est pas égale à 0 ; de préférence, la concentration masse-volume dudit tensioactif va de 0,2 mg/ml à 0,5 mg/ml.

10. La composition de facteur de croissance nerveuse selon l'une quelconque des revendications 1 à 9, **caractérisée en ce que** ledit tensioactif est un tensioactif non ionique, de préférence ledit tensioactif est au moins l'un parmi le poloxamère, le polysorbate et le 15-polyéthylèneglycol hydroxystéarate.

11. La composition de facteur de croissance nerveuse selon l'une quelconque des revendications 1 à 10, **caractérisée en ce que** dans ladite composition de facteur de croissance nerveuse :
la concentration masse-volume dudit facteur de croissance nerveuse est de 50 µg/ml ;
la concentration masse-volume dudit stabilisant est de 10 mg/ml ;
la concentration masse-volume dudit agent de support est de 40 mg/ml ;
la concentration molaire dudit tampon de pH est de 25 mM.

12. La composition de facteur de croissance nerveuse selon l'une quelconque des revendications 1 à 11, **caractérisée en ce que** ledit facteur de croissance nerveuse est choisi parmi un facteur de croissance nerveuse dérivé de la souris, un facteur de croissance nerveuse dérivé de l'humain ou un facteur de croissance nerveuse humain recombinant.

13. Un procédé de préparation d'une composition de facteur de croissance nerveuse sous la forme d'une poudre pour injection, **caractérisé en ce que** la composition de facteur de croissance nerveuse selon l'une quelconque des revendications 1 à 12 est lyophilisée pour obtenir ladite composition de facteur de croissance nerveuse sous la forme d'une poudre pour injection.

14. Une composition de facteur de croissance nerveuse sous la forme d'une poudre pour injection préparée par le procédé selon la revendication 13.

15. Une composition de facteur de croissance nerveuse selon l'une quelconque des revendications 1 à 14 pour une utilisation dans le traitement d'une lésion nerveuse, la lésion nerveuse pouvant être une lésion du nerf optique.
